# EUROPEAN PATENT SPECIFICATION

(11) **EP 4 149 659 B1**
(45) Date of publication and mention of the grant of the patent: **03.07.2024**
(21) Application number: 21724313.8
(22) Date of filing: 11.05.2021
(51) Int. Cl.: B01D 53/32, A61M 16/01, B01D 53/04, A61M 16/22

(54) **BREATHING SYSTEM DEVICE FOR CO2 REMOVAL BASED ON AN ELECTRO-CHARGING AND DISCHARGING METHOD**
BEATMUNGSSYSTEMVORRICHTUNG ZUR CO2-ENTFERNUNG AUF BASIS EINES ELEKTROLADE- UND ENTLADEVERFAHRENS
DISPOSITIF DE SYSTÈME RESPIRATOIRE POUR L'ÉLIMINATION DE CO2 SUR LA BASE D'UN PROCÉDÉ D'ÉLECTROCHARGE ET D'ÉLECTRODÉCHARGE

(30) Priority: 11.05.2020 BE 202005319
(43) Date of publication of application: 22.03.2023
(73) Proprietor: BRAEM, Kristof, 9320 Nieuwerkerken - Aalst (BE)
(72) Inventor: BRAEM, Kristof, 9320 Nieuwerkerken - Aalst (BE)
(74) Representative: Arnold & Siedsma
(86) International application number: PCT/EP2021/062530
(87) International publication number: WO 2021/228890

(56) References cited:
- US-A1- 2008 202 341
- US-A1- 2010 180 889
- US-A1- 2010 258 117
- US-A1- 2013 122 382

## Description

### Technical field

The invention generally relates to the field of anaesthesia, and in particular to purification of expiration gases during anaesthesia. More in particular, the invention relates to a regenerative device for CO2 removal, and to methods of regeneration of such a device for multiple use thereof. CO2 is removed from a (potential patient) gas stream, suitable for use in a breathing system, and for example being part of an anaesthesia arrangement. The invention is related to holders, containers and so-called canisters including any subparts of such devices and methods of operating used in an anaesthesia arrangement, wherein the CO2 removal takes place.

### Background of the invention

Anaesthesia ventilation is a closed or semi-open to closed system. This means that part of the ventilation exhaled by the patient will be reused for inspiration and ventilation of the patient. When reusing these gasses, the CO2 exhaled by the patient, has to be removed from the system.

Currently and mainly applied CO2 removing technique is based on the use of soda lime as an absorbent for CO2. Although recently, there is a tendency to move away from this technique because of the drawbacks of chemical absorbers, including for example the production of compounds being harmful to patients leading to increased cost and environmental impact, as well as the daily disposal of compound special waste. Moreover, the disposal of chemical granulates is becoming a growing environmental concern. As a sustainable alternatively, a membrane technique to filter CO2 from anaesthetic circuits can be used. This rather new technique relying on a polymeric membrane core for selectively allowing CO2 to leave the breathing system, while maintaining anaesthetic vapor in the circuit, is for the moment still more in experimental phase.

The use of soda lime, as an absorbent of the CO2, makes rebreathing possible, thus conserving gases and volatile agents, decreasing of pollution, and avoiding hazards of carbon dioxide rebreathing. Soda lime activator is NaOH or KOH. Silica and kieselguhr are added as hardeners. Indicators for soda lime (such as ethyl violet) are colourless when fresh, and purple when exhausted, because of pH changes in the granules. As a result, soda lime granules will change colour (to purple) when exhausted, being an indication (when purple) that they need to be replaced. Typically, every 2 days, one has to replace the soda lime with new granules.

The use of soda lime has many disadvantages, such as for example their reaction with other gasses. E.g. sevoflurane is unstable in soda lime, producing Compound A (lethal at 130-340 ppm, or renal injury at 25-50 ppm in rats; but incidence of toxic [hepatic or renal] or lethal effects in millions of humans are comparable to desflurane). Compound A concentrations of 25-50 ppm are easily achievable in normal clinical practice. Sevoflurane package insert recommends avoiding FGF at 1 L/min for no more than 2 MAC-Hours (2 L/min FGF can be used indefinitely). Further, carbon monoxide is produced by (desflurane > enflurane > isoflurane) >> (halothane = sevoflurane), whereas the situation being worse in dry absorbent. It is thus important to turn oxygen off at end of a patient case, change the absorbent regularly, e.g. change if FGF left on over the weekend or overnight, and use low flows, which will tend to keep granules moist.

Special and more expensive soda lime is already available on the market, to avoid the compound A production. However, still small part of the anaesthetic agent will react, resulting in a higher consumption than usually needed. The strong bases (NaOH, KOH which function as activators) have been convincingly implicated in the carbon monoxide problem with the ethyl-methyl ethers, and the generation of Compound A by sevoflurane, leading to very often replacement. Hence, waste management and the degree pollution have been considered. As a conclusion, current available solutions are rather expensive when calculated total cost per year/per unit.

In addition, other problems that can occur with soda lime are for example, the challenge in sizing a compromise between absorptive capacity and resistance to airflow, and the resistance of a full canister being < 1 cm H2O at 60 L/min flow through the canister. Further, inhaled dust is caustic and irritant. Moreover, one may see exhaustion without colour change, due to channelling or inactivation of indicator along the canister walls by UV light.

In general, in present devices for CO2 removal in anaesthetic arrangements as described above, soda lime granules are used, being removed and disposed of after their use. From an environmental point of view, such approach is no longer acceptable.

However, considering alternative more environmental friendly techniques for CO2 removal for use in the anaesthetic field is hampered by the observation that this field has specific requirements in terms of bio-compatibility for use with humans, that reusability requires autoclavability and one should consider particular use requirements in terms of typical (up to low) concentrations of CO2 in the gas stream and pressure restrictions when typical flow rate for this field application is used. Removal of gasses of a particular kind in an anaesthesia breathing context are known like in US2010258117 related to recycling of Xenon.

While US2008202341 emphasizes the importance of carbon dioxide removal in general and anaesthesia in particular, its use of an ionic membrane and/or by liquid impregnated cathode and anode makes it inappropriate for the particular use of anaesthesia. Carbon dioxide removal is also referred to in US2010180889, going along with oxygen generation for use in battlefield applications where oxygen requirements may be extreme.

### Aim of the invention

It is the aim of the invention to provide an environmentally friendly technique for CO2 removal for and adapted to the particular anaesthetic field.

### Summary of the invention

In a first aspect of the invention a device for CO2 removal from a gas stream, in particular an expiration or breathing gas stream (in the presence of an anaesthetic agent), suitable for use in a breathing system, part of an anaesthesia arrangement, is provided, as set forth in claim 1. The device comprises a holder, adapted for fitting in such breathing system, and a container for CO2 removal from a gas stream, wherein the container is adapted for fitting into the holder. According to an embodiment, the device consists of a holder, adapted for fitting in such breathing system, and a container for CO2 removal from a gas stream, wherein the container is adapted for fitting into the holder. The CO2 removal of the container is based on an electro-charging and discharging method. Whenever the device is placed in the breathing system, and the charging mode of the device is switched on, CO2 will be removed from the gas stream and will be captured and absorbed by the container. After a while, the container gets saturated with CO2 and has to be switched off for charging and switched on to discharging mode. This way the CO2 can be repelled from the container until it is free of CO2 again, and ready for charging reuse in the breathing system. The device is again operational for removing CO2 from the gas stream when switched on in charging mode. This iterative process can be repeated again.

The container comprises a plurality of plates, each comprising of at least two electrodes arranged for charging and/or discharging said plates. The plurality of plates can be adapted for absorbing CO2 when the electrodes being charged, and for releasing CO2 when the electrodes being discharged. The electrodes may comprise of an active electrode, and a counter electrode, being all or not separated by a dielectric or isolating separator. The device may comprise means for providing electricity to the active and/or counter electrode. Further, the device may comprise electronic means such as an electronic circuit to thereby control the charging and/or discharging operation of the electrodes. According to an embodiment, the device including holder, container, plates and electrodes are autoclavable and biocompatible for use with humans.

The device comprises at least two plates and may be adapted for having a pressure less than 2 mbar at a nominal flow rate between 20 to 65L/min, wherein the distance d between the plates the electrodes thereof is selected therefore.

According to an embodiment, the surface of the electrodes, i.e. the active electrode in particular, is selected to react with CO2 in the gas stream. The surface of the electrodes may be covered with a polymer, arranged in a design for optimal surface use like a honeycomb structure, wherein the polymer is containing material for attracting CO2 such as for example (anthra)quinone. The surface of the electrodes may be covered with a polymer, arranged in a design for optimal surface use like a honeycomb structure, wherein the polymer is comprising material for enhancing the conductivity such as for example carbon nanotubes.

According to an embodiment, the device is adapted for being capable to being operable for concentrations as low as around 400 ppm of CO2, by selecting of the amount of (anthra)quinone and the total surface (for CO2 removal) applicable in the container. This can be achieved depending on the of amount of (anthra)quinone and the total surface (for CO2 removal) applicable in the canister. The device can also be adapted for being operable for at least one day or 24 hours, preferably one (working) week of 5-7 days, wherein the operability in time can be chosen, designed or selected by means of the amount of plates used in the container for CO2 removal. Furthermore, the device can be adapted for being operable for at least 2000 charging-discharging cycles, preferably 5000 charging-discharging cycles with less than 30% efficiency loss of the charging-discharging operation.

The device for CO2 removal is designed via its selection of materials for CO2 absorbance, the amount of those materials used and the arrangement thereof, to be capable to handle typical (low) concentrations and/or to have a suitable cycle of use (for this application) while (given the environmental goal) also a certain durability. For instance, the amount of plates, and the amount of a (specific) polymer (provided with additional elements) may play a role in the design of the CO2 removal device, more in particular regarding the CO2 absorption capacity thereof. The design of the plate or electrode surface onto which a polymer is applied, may further be of importance. Moreover, in relation to the use requirements, the arrangement of the materials (for instance in terms of distance between plates) takes into account conditions on experienced pressure (by the patient).

In a second aspect of the invention a two-mode method for operating the device for CO2 removal in accordance with first aspect, is provided. The two-mode method comprises an electro-charging and discharging method for operating the device for CO2 removal. The comprises of the following steps. Firstly, when the device is installed in the gas flow circulation of a breathing system, part of an anaesthesia arrangement, from which it should remove the CO2, electricity of a first polarity is provided to the container in order to enable charging operation, meaning CO2 is captured and absorbed by the container under electricity. Secondly, when the device is removed from the gas flow circulation, electricity of a second polarity (different or reverse of the first policy) is provided to the container such that the device can be discharged, meaning CO2 is repelled or removed again from the container under electricity.

### Brief description of the drawings

Figure 1 illustrates schematically an embodiment of a device comprising an electrochemical plate and corresponding electrodes for CO2 removal from a gas stream, suitable for use in a breathing system, part of an anaesthesia arrangement, not in accordance with the invention.
Figures 2 shows a further embodiment of a device comprising two electrochemical plates with respective electrodes for CO2 removal from a gas stream, suitable for use in a breathing system, part of an anaesthesia arrangement, in accordance with the invention.
Figure 3 illustrates a more detailed version of the embodiment of Figure 1 including means to provide electricity to the CO2 removal device.
Figure 4 illustrates an exemplary embodiment of (a) charging, and (b) discharging process of a CO2 removal device with a plate defined by electrodes and separators there in between, and wherein means for providing electricity to the electrodes are foreseen, in accordance with the invention.
Figure 5 illustrates an embodiment of the use of the CO2 removal device in a breathing system context, in accordance with the invention.

### Detailed description of the invention

With the present invention, a traditional soda lime canister is replaced by a canister, holder or device containing a stack of electrochemical plates absorbing carbon dioxide from the circuit breathing gasses (or other gas stream) passing over its electrodes or e.g. a stack of electrodes, as the electrodes of the plates are charged up, and then releasing the CO2 gas as the electrodes of the plates are discharged. Hence, a new way of removing CO2 in the breathing system (wherein a stream of rebreathing gasses of the patient and/or fresh gas mixture is circulated) of an anaesthesia arrangement or workstation, is provided by means of an electro-charging and discharging system. According to an embodiment, CO2 is removed from a gas mixture of air, oxygen, nitrous oxide and/or anaesthetic agent (isoflurane, enflurane, desflurane, halothane and sevoflurane). This mixture may be either as combination of these different gasses, as well as in combination with individual gasses.

While charging, an electrochemical reaction takes place at the surface of the electrodes (or each of a stack of electrodes) and will attract the CO2 in the breathing circuit, meaning that the electrodes (or the surfaces thereof) will capture and absorb the CO2 present in the gas stream passing by. The electrodes may have a natural affinity for carbon dioxide and may readily react with its molecules in the gas stream, even when it is present a very low CO2 concentrations (down to the roughly 400 parts per million currently found in the atmosphere). The whole system operates at room temperature and normal pressure. The electrodes can be coated with a polymer, containing (anthra)quinone and composited with carbon nanotubes. The reverse reaction will take place while discharging.

According to an embodiment, the electrochemical plates comprise of one or more active coated electrodes, one or more counter electrodes and a separator between each active and counter electrode respectively. In an embodiment, the active electrodes and counter electrodes are placed or separated far enough from each other, such that a separator in between them is no longer necessary. In an embodiment, the complete electrochemical cell consists of an active electrode (or stack of electrodes), a counter electrode and/or a separator (depending on mounting position). By means of example, a graphene sheet can be used as counter electrode. The system can work at virtually any CO2 concentration level, even down to the roughly 400 parts per million currently found in the atmosphere.

The canister containing the electrodes can be placed or located in the breathing system on the inspiratory side, as well as the expiratory side.

The number of electrode plates or the total electrode surface (e.g. given by a stack of electrodes) used will determine the capacity of the CO2 absorbing. According to an embodiment, the distance/openings between the different plates (or 1 plate in the form of honeycomb) is between 0,8mm and 5,4mm.

With the present invention, a reusable system for CO2 removal can be provided for at least 2000 charging-discharging cycles, with less than 30% efficiency loss of that time. The full assembly of the system, meaning all parts, subparts including the materials they are made of, being not only reusable, but is moreover autoclavable and biocompatible for use with humans. By means of example, materials such as Makralon 2458, and Valox Resin HX420HP are applicable. There is no need of daily or weekly replacement, as the system can be charged and discharged for e.g. 5000 charging-discharging cycles with less than 30% efficiency loss. Having such amount of charging-discharging cycles, the system in accordance with the invention is much more sustainable, and has a significant life time as compared to the art.

The electrodes or stack of electrodes can be mounted in a container or canister, with sufficient distance between the electrodes, such that the breathing gasses can pass without causing more than 1cm H2O pressure at a flow of 60 litres/min.

The canister or container for CO2 removal can be placed in the circle system (on inspiratory side or expiratory side), as an add on by means of e.g. 22mm Male connector output, and 22mm Female connector input. The CO2 removal system in accordance with the invention can also be mounted into the existing absorber canister of the respective manufacturer.

According to an embodiment, an existing canister of anaesthesia arrangement or workstation can be used (or new one made compatible), comprising the CO2 removal system in accordance with the invention, such that this CO2 removal system can be connected at same position of current soda lime canister.

### Detailed description of the drawings

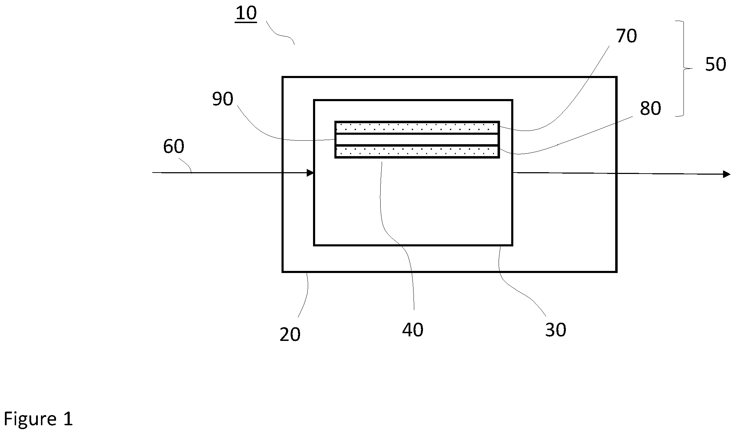

Figure 1 illustrates schematically a device 10 for CO2 removal from a gas stream 60, suitable for use in a breathing system, part of an anaesthesia arrangement. The device comprises a holder 20, adapted for fitting in the breathing system and a container 30 for CO2 removal from a gas stream 60, adapted for fitting in said holder. The container 30 removes CO2 from the gas stream 60 passing through, based on an electro-charging and discharging method. For enabling this, the container 30 in Figure 1 comprises a plate 40, e.g. an electrochemical plate, comprising of electrodes 50, amongst which for example an active electrode 70 and a counter electrode 80, being separated from each other by means of a dielectric or isolating separator 90. The charging of the device 10 for absorbing CO2 from the gas steam 60 takes place when charging the active electrode 70 by means of providing electricity there through. According to an embodiment, the surface of the active electrode 70 is provided with material, e.g. a polymer, for attracting CO2. For the electricity provided, one may use for instance voltages of 1-3V. Discharging of the device 10 occurs when the electricity going through the active electrode changes polarity, and thus the CO2 flow is reversed, i.e. instead of CO2 being attracted by the active electrode 70, the CO2 is now being repelled.

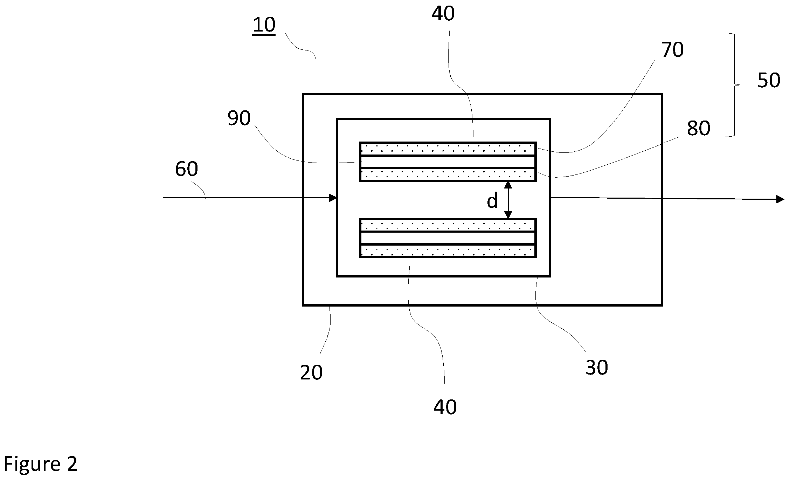

Figures 2 shows a further embodiment of the device of Figure 1, wherein two plates 40 comprising electrodes 50 are drawn, adapted for absorbing CO2 when the device 10 being charged and releasing CO2 when the device 10 is discharged. Between the plates 40, or the electrodes 50 thereof, a distance d is provided. The distance d between the plates 40 plays a role in relation to the pressure requirements for given nominal rates of the gas stream 60 passing between the plates 40, or the electrodes 50 thereof. The distance d between the plates 40 is for example 2mm, with a minimum of 14mm total distance available within the container 30 or canister. According to an embodiment (not shown), by means of example 6 to 8 plates 40 could be provided at 2mm distance from each other within a single container 30. Pressure resistance on the inspiratory side of the canister, may be up to 0,9cm H2O at peak flow between 20 to 65 litres a minute. With active canister in the breathing system, resistance test of the complete system, including the inventive canister, complies to MDR standards (total maximum resistance lower than 6cm H2O). The canister can be put on the inspiratory side of the circle anaesthesia rebreathing system, as well as on the expiratory side. Total content of the canister may be for example 1,8 litre (but can be changed to other sizes as well). Pressure in the circuit (and the absorber device) may be between 0 to 120cm H2O, without any influence on the functionality of the CO2 absorption. The complete canister set is autoclavable, e.g. 134°c steam sterilization, 8 minutes cycle.

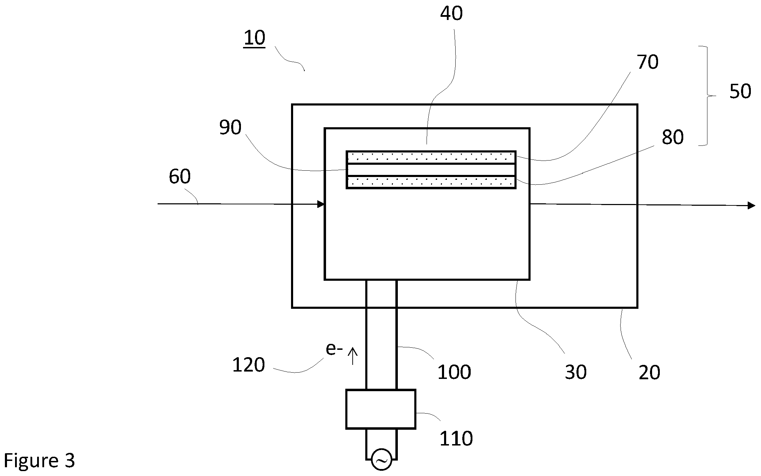

Figure 3 shows schematically the electricity 120 being provided to the device 10 of Figure 1, using means 100 being for example an electrically conducting cable or wire. The means 100 is on one hand connected to the container 30 within the holder 20 of the device 10. More in particular (not shown), the means 100 is connected to the electrodes 50, e.g. the active electrode 70 of the plate 40 for charging the electrodes 50 thereof. On the other hand, the means 100 is connected to a power supply, and an electronic means 110 such as an electronic circuit including e.g. an on-off switch for controlling the electro-charging operation of the device 10.

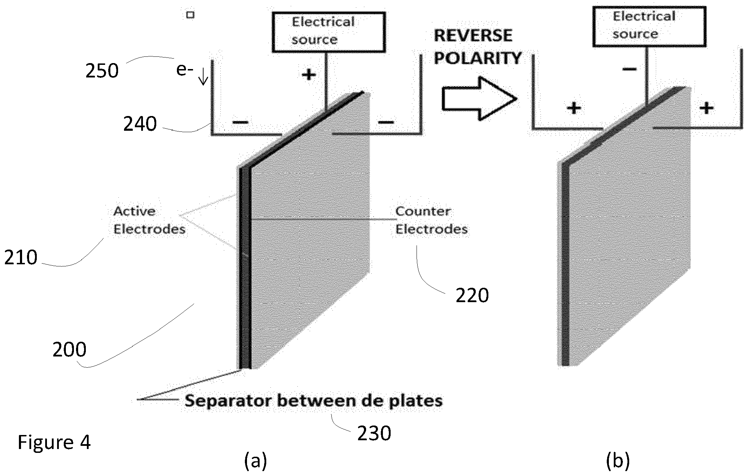

Figure 4 illustrates an exemplary embodiment of part of the device for CO2 removal in accordance with the invention. A plate 200 is defined by electrodes, in particular an active electrode 210, on the outer side of the plate 200, and a counter electrode 220, on the inner side of the plate 200. The electrodes 210, 220 are separated from each other by a separator 230 having an electrical isolating function. Means 240 e.g. electrical cables or wires, for providing electricity 250 to the active electrodes 210 and/or the counter electrode 220 are foreseen. Figure 4 also illustrates the two modes of operating for the embodiment wherein in a first mode, depicted in Figure 4 (a) one provides electricity of a first polarity to charge, and thus removing CO2 from the gas stream wherein the device is immersed, while the CO2 is captured by the active electrodes 210 of the plate 200 and for example being absorbed onto their outer surfaces. The first polarity is indicated by means of the arrow next to e-. A second mode is depicted in Figure 4 (b). The device is now removed from the gas stream, as for instance being saturated by CO2, and hence ready to be discharged out of the breathing system. Here electricity 250 is provided of a second polarity being different or reverse of the first policy, as indicated by means of reverse arrow next to e-. With this second polarity of electricity 250, the device is now discharged and hence CO2 stored in the device (as being captured thereby) is being released.

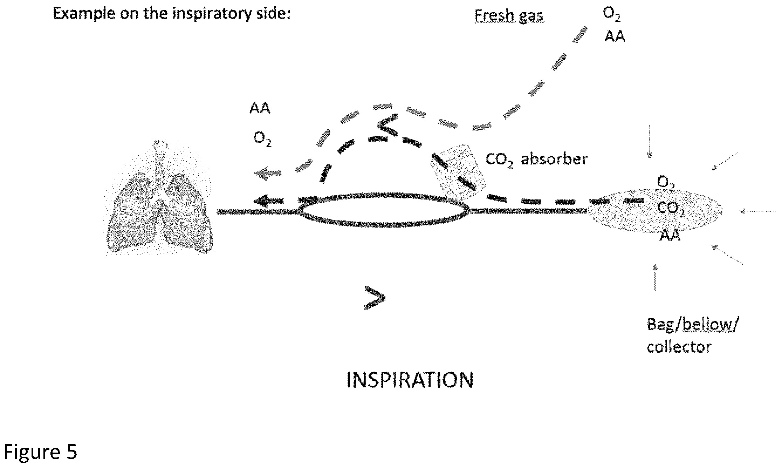

Figure 5 illustrates the use of the invention in a breathing system context. It is worth emphasizing that the holder or container, also called canister in the field, is or can be provided with suitable connectors of different types (male, female) with typical dimensions of 22 mm diameter. According to this embodiment, the CO2 removal device is placed at the inspiratory side, although it could also be provided at the expiratory side.

According to an embodiment (not shown) the breathing system comprises of at least two CO2 removal devices in accordance with the invention. While one of those devices is switched on for charging, the other one could simultaneously be switched off for charging, and e.g. switched on for discharging. This way, the containers or canisters can alternatingly charge and discharge in parallel, and in a synchronous manner, meaning while one is charging, the other one is discharging at the same time. This time could be for instance one day (or 24 hours) while also depending on the capacity of the canister for CO2 absorption, and hence depending on the amount of CO2 concentration present or captured by the canister after one day. Moreover, the more CO2 in the breathing system, the more needs to and will be captured and absorbed by the CO2 removal device.

It is further noted that, in case only one single CO2 removal device is used in the breathing system, the operational settings may be such that discharging goes faster than charging. In other words, it would probably be desirably then, that the charging on-status of the CO2 removal device is much longer than the off-status when discharging can take place, and hence not much time is lost or spent during the discharging operation.

## Claims

1. A device (10) for CO2 removal from an expiration or breathing gas stream (60), suitable for use in a breathing system, part of an anaesthesia arrangement, the device comprising: a holder (20), adapted for fitting in such breathing system, and a container (30) for CO2 removal from a gas stream (60), adapted for fitting in said holder, wherein said container being based on an electro-charging and discharging method, wherein said container (30) comprises a plurality of plates (40), arranged in that the expiration or breathing gas stream (60) passes between the plates (40), said plates (40) each comprising of at least two electrodes (50) arranged for charging and/or discharging said plates (40) and wherein said electrodes (50) comprise of an active electrode (70), and a counter electrode (80), being separated by a dielectric or isolating separator (90), and said device comprising means (100) for providing electricity (120) to said active and/or said counter electrode (70, 80).

2. The device (10) of claim 1 wherein said plurality of plates (40) being adapted for absorbing CO2 when said electrodes (50) being charged, and releasing CO2 when said electrodes (50) being discharged.

3. The device (10) of claim 1 or 2, further comprising electronic means (110) to thereby control charging or discharging of said electrodes (50).

4. The device (10) of claim 1 to 3, adapted for having a pressure less than 2 mbar at a nominal flow rate between 20 to 65L/min, and comprising at least two plates (40), wherein the distance d between said plates (40) or said electrodes (50) thereof is selected therefore.

5. The device (10) of claim 1 to 4, wherein the surface of said electrodes being selected to react with CO2 in said gas stream (60).

6. The device (10) of claim 5, wherein the surface of said electrodes being covered with a polymer, containing material for attracting CO2 such as (anthra)quinone.

7. The device (10) of claim 5 or 6, wherein the surface of said electrodes being covered with a polymer, comprising material for enhancing the conductivity such as carbon nanotubes.

8. The device (10) of claim 6, adapted for being capable to being operable for concentrations as low as around 400 ppm of CO2, by selecting of the amount of material for attracting CO2 such as (anthra)quinone and the total surface applicable in the container.

9. The device (10) of any of the previous claims, adapted for being operable for at least one day, preferably one week, by means of the amount of plates used in the container for CO2 removal.

10. The device (10) of any of the previous claims, adapted for being operable for at least 2000 charging-discharging cycles, preferably 5000 charging-discharging cycles with less than 30% efficiency loss of said charging-discharging operation.

11. A breathing system, comprising the device (10) for CO2 removal from a gas stream (60) in accordance with claim 1 to 10.

12. An anaesthesia arrangement, comprising the breathing system of claim 11.

13. An electro-charging and discharging method for operating the device (10) for CO2 removal from an expiration or breathing gas stream (60) in accordance with claim 1 to 10, comprising of the steps of: (i) when said device (10) is installed in the gas flow circulation of a breathing system, part of an anaesthesia arrangement, from which it should remove the CO2, providing electricity of a first polarity to said container (30) to charge; and (ii) when said device (10) is removed from said gas flow circulation, providing electricity of a second polarity (different or reverse of said first policy) to said container (30) to discharge.

14. A discharging method for operating the device (10) for CO2 removal from an expiration or breathing gas stream (60) in accordance with claim 1 to 10, comprising the step of removing said device (10) from the gas flow circulation, of a breathing system, part of an anaesthesia arrangement, from which it should remove the CO2, and providing electricity of a polarity to said container (30) to discharge.

## Patentansprüche

1. Vorrichtung (10) für eine CO2-Entfernung aus einem Ausatmungs- oder Atemgasstrom (60), die zur Verwendung in einem Atmungssystem, ein Teil einer Anästhesieanordnung ist, geeignet ist, die Vorrichtung umfassend: einen Halter (20), der angepasst ist, um in ein derartiges Atmungssystem zu passen, und einen Behälter (30) für die CO2-Entfernung aus einem Gasstrom (60), der angepasst ist, um in den Halter zu passen, wobei der Behälter auf einem Elektroauflade- und -entladeverfahren basiert, wobei der Behälter (30) eine Vielzahl von Platten (40) umfasst, die so angeordnet sind, dass der Ausatmungs- oder Atemgasstrom (60) zwischen den Platten (40) hindurchgeht, die Platten (40) jeweils umfassend mindestens zwei Elektroden (50), die zum Laden und/oder Entladen der Platten (40) angeordnet sind, und wobei die Elektroden (50) eine aktive Elektrode (70) und eine Gegenelektrode (80) umfassen, die durch einen dielektrischen oder isolierenden Separator (90) getrennt sind, und die Vorrichtung umfassend Mittel (100) zum Bereitstellen von Elektrizität (120) an die aktive und/oder die Gegenelektrode (70, 80).

2. Vorrichtung (10) nach Anspruch 1, wobei die Vielzahl von Platten (40) angepasst sind, um CO2 zu absorbieren, wenn die Elektroden (50) geladen werden, und CO2 abzugeben, wenn die Elektroden (50) entladen werden.

3. Vorrichtung (10) nach Anspruch 1 oder 2, ferner umfassend elektronische Mittel (110), um dadurch das Laden oder Entladen der Elektroden (50) zu steuern.

4. Vorrichtung (10) nach Anspruch 1 bis 3, die angepasst ist, um einen Druck von weniger als 2 mbar bei einer Nennströmungsrate zwischen 20 und 65 L/min aufzuweisen, und umfassend mindestens zwei Platten (40), wobei der Abstand d zwischen den Platten (40) oder den Elektroden (50) davon deshalb ausgewählt ist.

5. Vorrichtung (10) nach Anspruch 1 bis 4, wobei die Oberfläche der Elektroden ausgewählt ist, um mit CO2 in dem Gasstrom (60) zu reagieren.

6. Vorrichtung (10) nach Anspruch 5, wobei die Oberfläche der Elektroden mit einem Polymer bedeckt ist, das ein Material zum Anziehen von CO2, wie (Anthra)chinon, enthält.

7. Vorrichtung (10) nach Anspruch 5 oder 6, wobei die Oberfläche der Elektroden mit einem Polymer bedeckt ist, umfassend das Material zum Verbessern der Leitfähigkeit, wie Kohlenstoffnanoröhren.

8. Vorrichtung (10) nach Anspruch 6, die angepasst ist, um in der Lage zu sein, um für Konzentrationen so niedrig wie etwa 400 ppm CO2 betriebsfähig zu sein, durch Auswählen der Menge an Material zum Anziehen von CO2, wie (Anthra)chinon und die gesamte Oberfläche, die in dem Behälter anwendbar ist.

9. Vorrichtung (10) nach einem der vorstehenden Ansprüche, die angepasst ist, um für mindestens einen Tag, vorzugsweise eine Woche, mittels der Menge an Platten, die in dem Behälter für die CO2-Entfernung verwendet werden, betriebsfähig zu sein.

10. Vorrichtung (10) nach einem der vorstehenden Ansprüche, die angepasst ist, um für mindestens 2000 Auflade-Entlade-Zyklen, vorzugsweise 5000 Auflade-Entlade-Zyklen, mit einem Wirkungsgradverlust von weniger als 30 % des Auflade-Entlade-Betriebs betriebsfähig zu sein.

11. Atemsystem, umfassend die Vorrichtung (10) für die CO2-Entfernung aus einem Gasstrom (60) nach Anspruch 1 bis 10.

12. Anästhesieanordnung, umfassend das Atmungssystem nach Anspruch 11.

13. Elektroauflade- und -entladeverfahren zum Betreiben der Vorrichtung (10) für die CO2-Entfernung aus einem Ausatmungs- oder Atemgasstrom (60) nach dem Anspruch 1 bis 10, umfassend die Schritte: (i) wenn die Vorrichtung (10) in dem Gasströmungskreislauf eines Atmungssystems installiert ist, das Teil einer Anästhesieanordnung ist, aus der sie das CO2 entfernen soll, Bereitstellen von Elektrizität einer ersten Polarität dem Behälter (30), um aufzuladen; und (ii) wenn die Vorrichtung (10) aus dem Gasströmungskreislauf entfernt wird, Bereitstellen der Elektrizität einer zweiten Polarität (unterschiedlich oder umgekehrt zu der ersten Polarität) dem Behälter (30), um zu entladen.

14. Entladeverfahren zum Betreiben der Vorrichtung (10) für die CO2-Entfernung aus einem Ausatmungs- oder Atemgasstrom (60) nach Anspruch 1 bis 10, umfassend den Schritt des Entfernens der Vorrichtung (10) aus dem Gasströmungskreislauf eines Atmungssystems, ein Teil einer Anästhesieanordnung, aus dem sie das CO2 entfernen soll, und des Bereitstellens von Elektrizität einer Polarität dem Behälter (30), um zu entladen.

## Revendications

1. Dispositif (10) d'élimination de CO2 d'un flux de gaz d'expiration ou de respiration (60), pouvant être utilisé dans un système respiratoire faisant partie d'un dispositif d'anesthésie, le dispositif comprenant : un support (20), adapté pour être monté dans un tel système respiratoire, et un récipient (30) pour l'élimination de CO2 d'un flux gazeux (60), adapté pour être monté dans ledit support, dans lequel ledit récipient est basé sur un procédé d'électrocharge et de décharge, dans lequel ledit récipient (30) comprend une pluralité de plaques (40), agencées de manière à ce que le flux gazeux d'expiration ou de respiration (60) passe entre les plaques (40), lesdites plaques (40) comprenant chacune au moins deux électrodes (50) agencées pour charger et/ou décharger lesdites plaques (40) et dans lequel lesdites électrodes (50) comprennent une électrode active (70) et une contre-électrode (80) étant séparées par un séparateur diélectrique ou isolant (90), et ledit dispositif comprend des moyens (100) pour fournir de l'électricité (120) à ladite électrode active et/ou à ladite contre-électrode (70, 80).

2. Dispositif (10) selon la revendication 1 dans lequel ladite pluralité de plaques (40) sont adaptées pour absorber le CO2 lorsque lesdites électrodes (50) sont chargées, et pour libérer le CO2 lorsque lesdites électrodes (50) sont déchargées.

3. Dispositif (10) selon la revendication 1 ou 2, comprenant en outre des moyens électroniques (110) pour commander la charge ou la décharge desdites électrodes (50).

4. Dispositif (10) selon les revendications 1 à 3, adapté pour avoir une pression inférieure à 2 mbar à un débit nominal compris entre 20 et 65 L/min, et comprenant au moins deux plaques (40), dans lequel la distance d entre lesdites plaques (40) ou lesdites électrodes (50) est choisie en conséquence.

5. Dispositif (10) selon les revendications 1 à 4, dans lequel la surface desdites électrodes est sélectionnée pour réagir avec le CO2 dans ledit flux gazeux (60).

6. Dispositif (10) selon la revendication 5, dans lequel la surface desdites électrodes est recouverte d'un polymère contenant un matériau permettant d'attirer le CO2, tel que l'(anthra)quinone.

7. Dispositif (10) selon la revendication 5 ou 6, dans lequel la surface desdites électrodes est recouverte d'un polymère comprenant des matériaux destinés à améliorer la conductivité, tels que des nanotubes de carbone.

8. Dispositif (10) selon la revendication 6, adapté pour être capable de fonctionner pour des concentrations aussi faibles qu'environ 400 ppm de CO2, en sélectionnant la quantité de matériau pour attirer le CO2 tel que l'(anthra)quinone et la surface totale applicable dans le conteneur.

9. Dispositif (10) selon l'une quelconque des revendications précédentes, adapté pour fonctionner pendant au moins un jour, de préférence une semaine, grâce à la quantité de plaques utilisées dans le conteneur pour l'élimination du CO2.

10. Dispositif (10) selon l'une quelconque des revendications précédentes, adapté pour fonctionner pendant au moins 2000 cycles de charge-décharge, de préférence 5000 cycles de charge-décharge avec moins de 30 % de perte d'efficacité de ladite opération de charge-décharge.

11. Système respiratoire comprenant le dispositif (10) d'élimination de CO2 d'un flux gazeux (60) selon les revendications 1 à 10.

12. Dispositif d'anesthésie comprenant le système respiratoire de la revendication 11.

13. Procédé d'électrocharge et de décharge pour faire fonctionner le dispositif (10) d'élimination de CO2 d'un flux de gaz d'expiration ou de respiration (60) selon les revendications 1 à 10, comprenant les étapes suivantes : (i) lorsque ledit dispositif (10) est installé dans la circulation de gaz d'un système respiratoire, faisant partie d'un dispositif d'anesthésie, dont il doit extraire le CO2, la fourniture d'électricité de première polarité audit conteneur (30) pour le charger ; et (ii) lorsque ledit dispositif (10) est retiré de ladite circulation de gaz, la fourniture d'électricité d'une seconde polarité (différente ou inverse de ladite première politique) audit conteneur (30) pour le décharger.

14. Procédé de décharge pour faire fonctionner le dispositif (10) d'élimination de CO2 d'un flux de gaz d'expiration ou de respiration (60) selon les revendications 1 à 10, comprenant l'étape consistant à retirer ledit dispositif (10) de la circulation du flux de gaz, d'un système respiratoire, faisant partie d'un dispositif d'anesthésie, à partir duquel il doit éliminer le CO2, et à fournir de l'électricité d'une certaine polarité audit conteneur (30) pour le décharger.
